# EUROPÄISCHE PATENTSCHRIFT

(11) **EP 2 737 884 B1**
(45) Veröffentlichungstag und Bekanntmachung des Hinweises auf die Patenterteilung: **14.06.2017**
(21) Anmeldenummer: 12195277.4
(22) Anmeldetag: 03.12.2012
(51) Int. Cl.: A61F 9/007

(54) **Medizinische Hohlnadel**
Medical hollow needle
Aiguille creuse médicale

(43) Veröffentlichungstag der Anmeldung: 04.06.2014
(73) Patentinhaber: Carriazo, Cesar C., Dr., Baranquilla (CO)
(72) Erfinder: Carriazo, Cesar C., Dr., Baranquilla (CO)
(74) Vertreter: Hofstetter, Schurack & Partner

(56) Entgegenhaltungen:
- US-A- 4 643 717
- US-A- 4 959 049
- US-A1- 2012 197 215

## Beschreibung

Die vorliegende Erfindung betrifft eine medizinische Hohlnadel gemäß dem Oberbegriff des Anspruchs 1.

Derartige Hohlnadeln sind aus der US 4 959 049 A und der US 4 643 717 A bekannt.

Die als Katarakt (grauer Star) bezeichnete Eintrübung der Augenlinse ist heute eine sehr häufige Erkrankung, von der insbesondere ältere Menschen betroffen sind. Die einzige Möglichkeit der Korrektur ist die chirurgische Behandlung. Sie besteht in der Entfernung von vorderer Linsenkapsel, Linsenrinde und Linsenkern mit anschließender Implantation einer sog. Intraokularlinse (IOL), die in den Linsenkapselsack eingeführt wird und aus unterschiedlichen Materialien bestehen kann.

Die Augenlinse wird dabei durch eine so genannte Phakoemulsifikation entfernt. Dabei wird unter Einsatz von Ultraschall die Augenlinse zerstört und abgesaugt. Hierfür wird eine dünne medizinische Hohlnadel in die Augenlinse eingeführt, wobei die Hohlnadel von einem Ultraschallgenerator zum Vibrieren gebracht wird. Dadurch wird die Augenlinse in kleine Bruchstücke zerlegt, die dann über die medizinische Hohlnadel abgesaugt werden können. Zur Unterstützung der Zerkleinerung der Augenlinse wird über einen zweiten Schnitt üblicherweise ein zweites medizinisches Instrument in die Augenlinse eingeführt. Das Einführende dieses zweiten medizinischen Instruments kann dabei löffelartig oder hakenartig ausgebildet sein und unterstützt die Zertrümmerung der Augenlinse mittels der oszillierenden Hohlnadel. Insbesondere bei sehr harten Linsenkernen muss dieses zweite medizinische Instrument zur Unterstützung der vollständigen Zertrümmerung der Augenlinse eingebracht werden. Nachteilig hieran ist jedoch, dass es durch die Einführung von zwei Instrumenten in die Augenlinse zu einer vermehrten Belastung des Patienten kommt.

Aufgabe der Erfindung ist es daher eine medizinische Hohlnadel der eingangs genannten Art bereitzustellen, welche eine gesteigerte Effizienz und eine geringere Belastung eines Patienten bei einer Phakoemulsifikation gewährleistet.

Gelöst wird diese Aufgabe durch eine gattungsgemäße medizinische Hohlnadel mit den Merkmalen des Anspruchs 1.

Vorteilhafte Ausgestaltungen der medizinischen Hohlnadel sind in den Unteransprüchen beschrieben.

Eine erfindungsgemäße medizinische Hohlnadel umfasst mindestens eine Saugröhre oder Saugleitung mit einem körperfernen, proximalen Ende und einem körpernahen, distalen Ende, wobei das distale Ende als Saugöffnung ausgebildet ist. Dabei ist an der Saugöffnung am distalen Ende der Saugröhre oder der Saugleitung mindestens ein Vorsprung angeordnet, wobei der Vorsprung über einen Rand der Saugöffnung und das distale Ende der Saugröhre oder der Saugleitung hinausragend ausgebildet ist. Durch die Ausbildung von mindestens einem Vorsprung, der über den Rand der Saugöffnung und das distale Ende der Saugröhre oder der Saugleitung hinausragt, wird die Wirkung der bewegten Hohlnadel innerhalb der zu zertrümmernden Augenlinse deutlich erhöht. Der ebenfalls mitbewegte Vorsprung erhöht die Effizienz der Zertrümmerung der Augenlinse derart, dass kein zweites medizinisches Instrument zur Unterstützung der medizinischen Hohlnadel in die Augenlinse eingeführt werden muss. Dadurch verringert sich die Belastung des Patienten deutlich. Der Vorsprung ist dabei als separates Element ausgebildet und entsprechend mit dem distalen Ende der Saugröhre oder Saugleitung verbunden. Vorteilhafterweise kann der Vorsprung je nach Anforderung ausgetauscht und gewechselt werden.

In vorteilhaften Ausgestaltungen der erfindungsgemäßen medizinischen Hohlnadel ist der Vorsprung kreisförmig, fächerförmig, schaufelartig, löffelartig, dornartig, nadelartig oder hakenartig ausgebildet. Auch andere Formen sind denkbar. Zudem besteht die Möglichkeit, dass der Vorsprung in distaler Richtung verjüngend oder in distaler Richtung spitz zulaufend ausgebildet ist. Weitere Ausgestaltungsmöglichkeiten des Vorsprungs sehen mindestens eine Schneide am Vorsprung vor. Die jeweilige Form des Vorsprungs hängt unter anderem auch von dem Zustand der zu zertrümmernden Augenlinse ab. Je nach vorliegendem Härtegrad der Augenlinse kann eine für den operativen Eingriff optimierte Form des Vorsprungs ausgewählt werden. So kann zum Beispiel bei dem Vorliegen einer sehr harten Augenlinse der Vorsprung eine Schneide aufweisen, durch die der Zertrümmerungseffekt der schwingenden Hohlnadel deutlich erhöht wird.

In weiteren vorteilhaften Ausgestaltungen der medizinischen Hohlnadel besteht die Saugröhre oder Saugleitung und/oder der Vorsprung aus Metall, einer Metall-Legierung, aus Kunststoff, aus Keramik oder einer Kombination davon. Die Materialauswahl kann dabei entsprechend den medizinischen Anforderungen getroffen werden.

Des Weiteren besteht die Möglichkeit, dass die medizinische Hohlnadel im Bereich ihres distalen Endes mindestens eine in der Saugröhre oder Saugleitung ausgebildete zusätzliche Absaugöffnung aufweist. Neben der eigentlichen, am distalen Ende angeordneten Saugöffnung kann die zusätzliche Saugöffnung in der Wand des distalen Endes der Hohlnadel den Absaugeffekt deutlich erhöhen. Es können somit in vorteilhafter Weise die zertrümmerten Teile der Augenlinse rasch über die Saugröhre oder Saugleitung abgeführt werden. Dies verkürzt in ebenfalls vorteilhafter Weise die Dauer des chirurgischen Eingriffs und senkt somit die Belastung für den Patienten.

Ein weiterer Aspekt der vorliegenden Erfindung betrifft ein medizinisches Gerät umfassend eine medizinische Hohlnadel wie im Vorhergehenden beschrieben, sowie eine Absaugvorrichtung, welche im Bereich eines proximalen Endes der Saugröhre oder der Saugleitung mit der Saugröhre oder Saugleitung verbunden ist. Dadurch ist ein sicheres Absaugen der zertrümmerten Teile der Augenlinse gewährleistet.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Es zeigen
- Figur 1a: eine schematische Darstellung einer medizinischen Hohlnadel gemäß dem Stand der Technik;
- Figur 1b: eine schematische Darstellung einer medizinischen Hohlnadel gemäß dem Stand der Technik;
- Figur 2: eine schematische Darstellung eines distalen Endes einer erfindungsgemäßen medizinischen Hohlnadel gemäß einer ersten Ausführungsform;
- Figur 3: eine schematische Darstellung eines distalen Endes einer erfindungsgemäßen medizinischen Hohlnadel gemäß einer zweiten Ausführungsform;
- Figur 4: eine schematische Darstellung eines distalen Endes einer erfindungsgemäßen medizinischen Hohlnadel gemäß einer dritten Ausführungsform;
- Figur 5: eine schematische Darstellung eines distalen Endes einer erfindungsgemäßen medizinischen Hohlnadel gemäß einer vierten Ausführungsform;
- Figur 6: eine schematische Darstellung eines distalen Endes einer erfindungsgemäßen medizinischen Hohlnadel gemäß einer fünften Ausführungsform; und
- Figur 7: eine schematische Darstellung eines distalen Endes einer erfindungsgemäßen medizinischen Hohlnadel gemäß einer sechsten Ausführungsform.

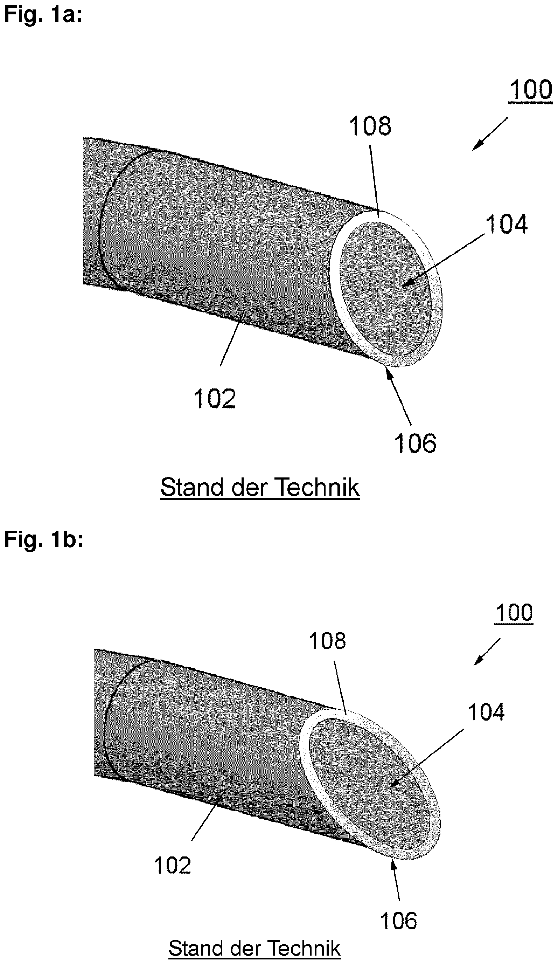

Die Figuren 1a und 1b zeigen in schematischen Darstellungen medizinische Hohlnadeln 100 gemäß dem Stand der Technik. Die bekannten medizinischen Hohlnadeln 100 werden ebenfalls mit einer Ultraschall erzeugenden Vorrichtung verwendet und bei der so genannten Phakoemulsifikation eingesetzt. Die Hohlnadeln 100 umfassen dabei eine Saugröhre 102, die an einem körpernahen, distalen Ende 106 eine Saugöffnung 104 ausbildet. Die Saugöffnung 104 ist von einem Rand 108 umgeben. In Figur 1a ist dieser Rand 108 rund ausgebildet, in Figur 1b ist das distale Ende 106 der medizinischen Hohlnadel 100 derart ausgeformt, dass der Rand 108 eine ovale Form aufweist.

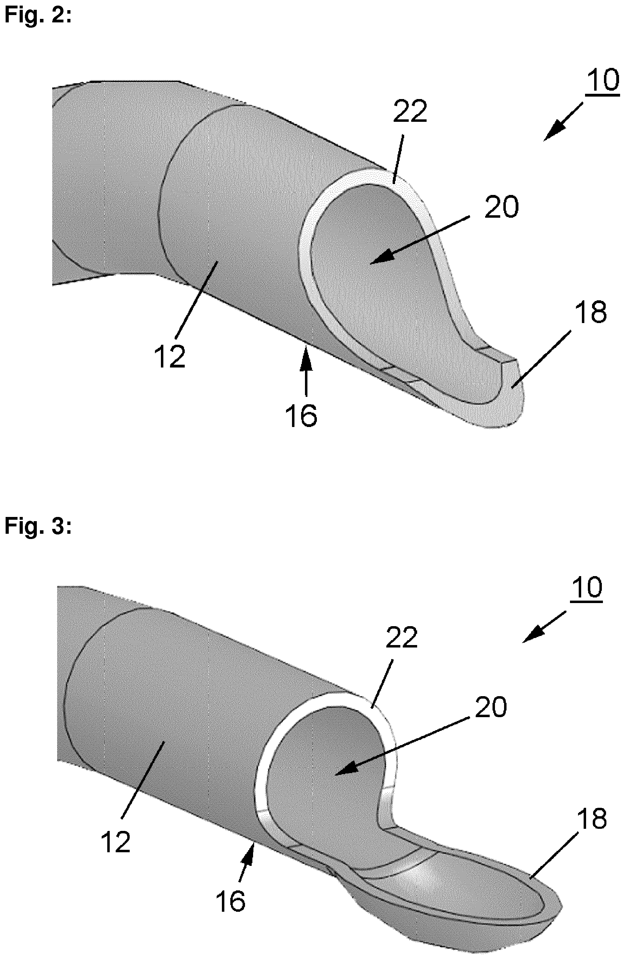

Figur 2 zeigt eine schematische Darstellung eines distalen Endes 16 einer medizinischen Hohlnadel 10 gemäß einer ersten Ausführungsform der Erfindung. Die Hohlnadel 10 ist dabei wiederum zur Verwendung mit einer Ultraschall erzeugenden chirurgischen Vorrichtung vorgesehen, wobei zumindest das distale Ende 16 der Hohlnadel 10 durch einen Ultraschallgenerator (nicht dargestellt) in Schwingungen versetzt wird. Die Hohlnadel 10 umfasst dabei eine Saugröhre bzw. Saugleitung 12 mit einem körperfernen, proximalen Ende (nicht dargestellt) und dem körpernahen, distalen Ende 16, wobei das distale Ende 16 als Saugöffnung 20 ausgebildet ist. Man erkennt, dass die Saugöffnung 20 gemäß dem ersten Ausführungsbeispiel der Hohlnadel 10 im Querschnitt ungefähr kreisförmig ausgebildet ist. Des Weiteren wird deutlich, dass an der Saugöffnung 20 am distalen Ende 16 der Saugröhre bzw. Saugleitung 12 ein Vorsprung 18 angeordnet ist. Der Vorsprung 18 ragt dabei über einen Rand 22 der Saugöffnung 20 und das distale Ende 16 der Saugröhre bzw. Saugleitung 12 hinaus. Der Rand 22 der Saugöffnung 20 ist dabei nicht durchgehend ausgebildet. Ein Teilbereich des Randes 22 wird durch den Vorsprung 18 vorgegeben bzw. ausgebildet. In dem dargestellten Ausführungsbeispiel weist der Vorsprung 18 eine schaufelförmige Ausgestaltung auf. Dabei ist ein Teilbereich der Saugröhre bzw. Saugleitung 12 über die Saugöffnung 20 hinaus verlängert. Der Vorsprung 18 ist mit der Saugröhre bzw. Saugleitung 12 einstückig ausgebildet.

Figur 3 zeigt eine schematische Darstellung des distalen Endes 16 einer medizinischen Hohlnadel 10 gemäß einer zweiten Ausführungsform. Im Gegensatz zu der in Figur 2 dargestellten ersten Ausführungsform ist hierbei der Vorsprung 18 löffelartig ausgebildet. Auch bei diesem Ausführungsbeispiel wird ein Teilbereich der Saugröhre bzw. Saugleitung 12 verlängert ausgebildet und formt den löffelartigen Vorsprung 18, der über die Saugöffnung 20 und das distale Ende 16 der Saugröhre bzw. Saugleitung 12 hinausragt.

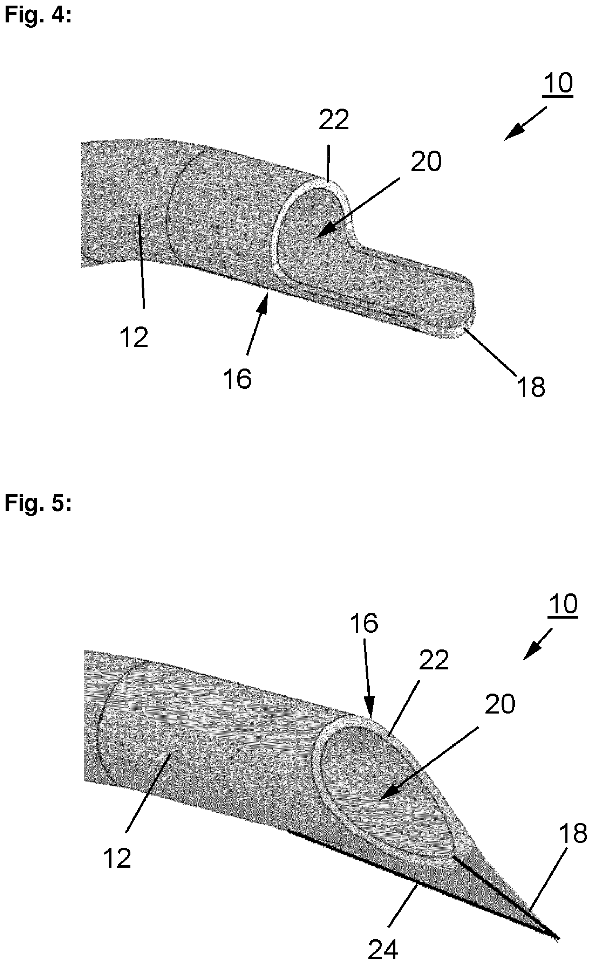

Figur 4 zeigt in einer schematischen Darstellung das distale Ende 16 einer medizinischen Hohlnadel 10 gemäß einer dritten Ausführungsform. Der Vorsprung 18 weist hierbei wiederum eine konkave, schaufelförmige Ausgestaltung auf. Allerdings ist im Gegensatz zu der in Figur 2 dargestellten Ausführungsform der Übergang zwischen dem Rand 22 der Saugöffnung 20 nicht abgeschrägt, sondern in einem Winkel von ungefähr 90° ausgebildet. Man erkennt deutlich, dass der Vorsprung 18 über den Rand 22 der Saugöffnung 20 und das distale Ende 16 hinausragend ausgebildet ist.

Figur 5 zeigt in einer schematischen Darstellung das distale Ende 16 einer medizinischen Hohlnadel 10 gemäß einer vierten Ausführungsform. Gemäß dieser Ausführungsform ist der Vorsprung 18 in distaler Richtung spitz zulaufend ausgebildet und weist eine Schneide 24 auf. Die Saugöffnung 20 weist in der Draufsicht eine ovale Form auf. Insgesamt ragt der Vorsprung 18 über den Rand 22 der Saugöffnung 20 und das distale Ende 16 der Saugröhre bzw. Saugleitung 12 hinaus. Man erkennt aber, dass ein Teilbereich des Vorsprungs 18 mit der daran angeordneten Schneide 24 in proximaler Richtung an der Saugröhre bzw. Saugleitung 12 anliegt bzw. damit ausgeformt ist.

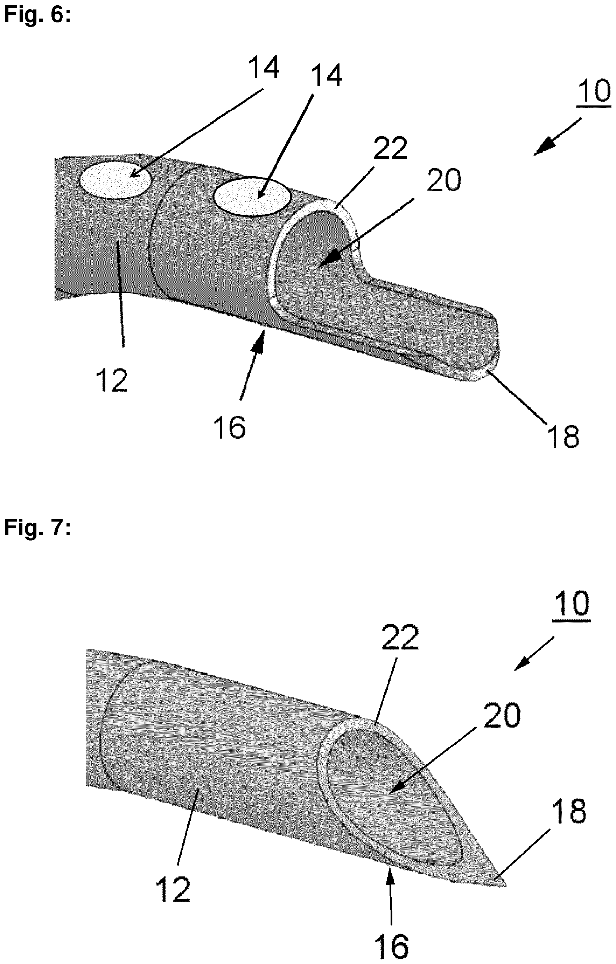

Figur 6 zeigt eine schematische Darstellung des distalen Endes 16 der medizinischen Hohlnadel 10 gemäß einer fünften Ausführungsform. Man erkennt, dass der Vorsprung 18 wie in dem in Figur 4 beschriebenen Ausführungsbeispiel ausgeformt ist. Im Gegensatz zu dem genannten Ausführungsbeispiel weist die medizinische Hohlnadel 10 gemäß dem fünften Ausführungsbeispiel im Bereich des distalen Endes 16 zwei in der Saugröhre bzw. Saugleitung 12 ausgebildete zusätzliche Absaugöffnungen 14 auf. Durch diese zusätzlichen Absaugöffnungen 14 erhöht sich die Saugleistung bzw. die Abflussrate durch die Saugröhre bzw. Saugleitung 12 erheblich.

Figur 7 zeigt eine schematische Darstellung des distalen Endes 16 der medizinischen Hohlnadel 10 gemäß einer sechsten Ausführungsform. Man erkennt, dass der Vorsprung 18 in distaler Richtung spitz zulaufend ausgebildet ist. Die Saugöffnung 20 weist in der Draufsicht eine ovale Form auf. Insgesamt ragt der Vorsprung 18 über den Rand 22 der Saugöffnung 20 und das distale Ende 16 der Saugröhre bzw. Saugleitung 12 hinaus.

Die im Vorhergehenden beschriebenen Ausführungsformen der medizinischen Hohlnadel 10 weisen jeweils Vorsprünge 18 auf, die mit der Saugröhre bzw. Saugleitung 12 einstückig ausgebildet sind. Dabei kann die Saugröhre bzw. Saugleitung 12 und der Vorsprung 18 aus Metall, einer Metall-Legierung, aus Kunststoff, aus Keramik oder einer Kombination davon bestehen. Im Bereich des proximalen Endes (nicht dargestellt) der Saugröhre bzw. Saugleitung 12 ist zudem jeweils eine Absaugvorrichtung (nicht dargestellt) mit der Saugröhre bzw. Saugleitung 12 verbunden.

## Patentansprüche

1. Medizinische Hohlnadel umfassend mindestens eine Saugröhre oder Saugleitung (12) mit einem körperfernen, proximalen Ende und einem körpernahen, distalen Ende (16), wobei das distale Ende (16) als Saugöffnung (20) ausgebildet ist, wobei an der Saugöffnung (20) am distalen Ende (16) der Saugröhre oder Saugleitung (12) mindestens ein Vorsprung (18) angeordnet ist und der Vorsprung (18) über einen Rand (22) der Saugöffnung (20) und das distale Ende (16) der Saugröhre oder der Saugleitung (12) hinausragend ausgebildet ist,
**dadurch gekennzeichnet,**
**dass** der Vorsprung (18) als separates Element ausgebildet ist und mit dem distalen Ende (16) der Saugröhre oder Saugleitung (12) verbunden ist.

2. Medizinische Hohlnadel nach Anspruch 1,
**dadurch gekennzeichnet,**
**dass** die Saugöffnung (20) im Querschnitt kreisförmig oder oval ausgebildet ist.

3. Medizinische Hohlnadel nach Anspruch 1 oder 2,
**dadurch gekennzeichnet,**
**dass** der Vorsprung (18) kreisförmig, fächerförmig, schaufelartig, löffelartig, dornartig, nadelartig oder hackenartig ausgebildet ist.

4. Medizinische Hohlnadel nach einem der vorhergehenden Ansprüche,
**dadurch gekennzeichnet,**
**dass** der Vorsprung (18) in distaler Richtung verjüngend oder in distaler Richtung spitz zulaufend ausgebildet ist.

5. Medizinische Hohlnadel nach einem der vorhergehenden Ansprüche,
**dadurch gekennzeichnet,**
**dass** der Vorsprung (18) mindestens eine Schneide (24) aufweist.

6. Medizinische Hohlnadel nach einem der vorhergehenden Ansprüche,
**dadurch gekennzeichnet,**
**dass** die Saugröhre oder Saugleitung (12) und/oder der Vorsprung (18) aus Metall, einer Metall-Legierung, aus Kunststoff, aus Keramik oder einer Kombination davon besteht.

7. Medizinische Hohlnadel nach einem der vorhergehenden Ansprüche,
**dadurch gekennzeichnet,**
**dass** die Hohlnadel (10) im Bereich ihres distalen Endes (16) mindestens eine in der Saugröhre oder der Saugleitung (12) ausgebildete zusätzliche Absaugöffnung (14) aufweist.

8. Medizinisches Gerät, umfassend eine medizinische Hohlnadel gemäß einem der vorhergehenden Ansprüche, sowie eine Absaugvorrichtung, welche im Bereich eines proximalen Endes der Saugröhre oder Saugleitung (12) mit der Saugröhre oder der Saugleitung (12) verbunden ist.

## Claims

1. Medical hollow needle comprising at least one suction tube or suction line (12) having a proximal end remote from the body and a distal end (16) close to the body, wherein said distal end (16) is formed as a suction aperture (20), wherein at said suction aperture (20) at said distal end (16) of said suction tube or suction line (12) at least one protrusion (18) is disposed and said protrusion (18) is formed to protrude beyond an edge (22) of said suction aperture (20) and said distal end (16) of said suction tube or suction line (12),
**characterized in that**
said protrusion (18) is formed as separate element and is connected to said distal end (16) of said suction tube or suction line (12).

2. The medical hollow needle of claim 1,
**characterized in that**
said suction aperture (20) is formed circularly or oval in cross-section.

3. The medical hollow needle of claim 1 or 2,
**characterized in that**
said protrusion (18) is formed circularly, fan-shaped, shovel-like, spoon-like, thorn-like, needle-like or hoe-like.

4. The medical hollow needle according to any one of the preceding claims,
**characterized in that**
said protrusion (18) is formed narrowing in the distal direction or tapering in the distal direction.

5. The medical hollow needle according to any one of the preceding claims,
**characterized in that**
said protrusion (18) comprises at least one blade (24).

6. The medical hollow needle according to any one of the preceding claims,
**characterized in that**
said suction tube or suction line (12) and/or said protrusion (18) consists of metal, a metal alloy, plastic, ceramic, or a combination thereof.

7. The medical hollow needle according to any one of the preceding claims,
**characterized in that**
the hollow needle (10) in the region of its distal end (16) comprises at least one additional suction aperture (14) formed in said suction tube or suction line (12).

8. Medical device, comprising a medical hollow needle according to any one of the preceding claims, as well as a suction device, which in the region of a proximal end of said suction tube or suction line (12) is connected with said suction tube or suction line (12).

## Revendications

1. Aiguille creuse médicale, comprenant au moins un tube d'aspiration ou une conduite d'aspiration (12) avec une extrémité proximale, éloignée du corps et une extrémité distale (16), proche du corps, l'extrémité distale (16) étant constituée en tant qu'orifice d'aspiration (20), au moins une saillie (18) étant agencée contre l'orifice d'aspiration (20), à l'extrémité distale (16) du tube d'aspiration ou de la conduite d'aspiration (12) et la saillie (18) étant constituée en surplomb par l'intermédiaire d'un bord (22) de l'orifice d'aspiration (20) et de l'extrémité distale (16) du tube d'aspiration ou de la conduite d'aspiration (12),
**caractérisée en ce**
**que** la saillie (18) est constituée en tant qu'élément séparé et est reliée à l'extrémité distale (16) du tube d'aspiration ou de la conduite d'aspiration (12).

2. Aiguille creuse médicale selon la revendication 1,
**caractérisée en ce**
**que** l'orifice d'aspiration (20) est constitué en coupe transversale de manière circulaire ou ovale.

3. Aiguille creuse médicale selon la revendication 1 ou 2,
**caractérisée en ce**
**que** la saillie (18) est constituée en forme de cercle, en forme d'éventail, en forme d'aube, en forme de cuillère, en forme de mandrin, en forme d'aiguille ou en forme de talon.

4. Aiguille creuse médicale selon l'une des revendications précédentes,
**caractérisée en ce**
**que** la saillie (18) est constituée, dans le sens distal, en se rétrécissant ou, dans le sens distal, en pointe.

5. Aiguille creuse médicale selon l'une des revendications précédentes,
**caractérisée en ce**
**que** la saillie (18) présente au moins un tranchant (24).

6. Aiguille creuse médicale selon l'une des revendications précédentes,
**caractérisée en ce**
**que** le tube d'aspiration ou la conduite d'aspiration (12) et / ou la saillie (18) se compose de métal, d'un alliage de métal, de plastique, de céramique ou d'une combinaison de ceux-ci.

7. Aiguille creuse médicale selon l'une des revendications précédentes,
**caractérisée en ce**
**que** l'aiguille creuse (10) présente, dans le secteur de son extrémité distale (16), au moins un orifice d'aspiration (14) supplémentaire, constitué dans le tube d'aspiration ou la conduite d'aspiration (12).

8. Appareillage médical, comprenant une aiguille creuse médicale conformément à l'une des revendications précédentes, ainsi qu'un dispositif d'aspiration, relié, dans le secteur d'une extrémité proximale du tube d'aspiration ou de la conduite d'aspiration (12), au tube d'aspiration ou à la conduite d'aspiration (12).
